# EUROPÄISCHE PATENTANMELDUNG

(11) **EP 4 759 269 A1**
(43) Veröffentlichungstag der Anmeldung: **17.06.2026**
(21) Anmeldenummer: 25217461.0
(22) Anmeldetag: 20.11.2025
(51) Int. Cl.: A61F 2/28, A61B 17/80, B33Y 80/00

(54) **IMPLANTAT FÜR EINE FLÄCHIGE BEHANDLUNG EINES KNOCHENDEFEKTS**

(30) Priorität: 11.12.2024 DE 102024137111
(71) Anmelder: Karl Leibinger Asset Management GmbH & Co. KG, 78570 Mühlheim an der Donau (DE)
(72) Erfinder: Aksu, Adem, 78054 VS-Schwenningen (DE); Schaarschmidt, Lena, 78532 Tuttlingen (DE)
(74) Vertreter: Schwamberger, Martin

(57) **Zusammenfassung**

Die Erfindung betrifft ein Implantat (I) für eine flächige Behandlung eines Knochendefekts, insbesondere eines Knochendefekts im Bereich des Thorax oder des Craniums, umfassend eine Gitterstruktur (GS) mit einer Oberseite (OS) und einer der Oberseite (OS) abgewandt liegenden Unterseite (US), auf welcher eine knochenseitige Befestigung des Implantats (I) vorzunehmen ist. Dabei weist die Gitterstruktur (GS) mindestens einen Tragabschnitt (TA) auf, welcher mittels eines additiven Fertigungsverfahrens hergestellt worden ist. Zudem ist auf den mindestens einen Tragabschnitt (TA) zumindest abschnittsweise jeweils eine Deckschicht (DS) aufgebracht, welche im Vergleich zu dem mindestens einen Tragabschnitt (TA) eine geringere Härte aufweist.

## Beschreibung

Die Erfindung betrifft ein Implantat für eine flächige Behandlung eines Knochendefekts, insbesondere eines Knochendefekts im Bereich des Thorax oder des Craniums, umfassend eine flexible Gitterstruktur mit einer Oberseite und einer der Oberseite abgewandt liegenden Unterseite, auf welcher eine knochenseitige Befestigung des Implantats vorzunehmen ist, wobei die Gitterstruktur mindestens einen Tragabschnitt aufweist.

Im Bereich der Implantate sind Varianten bekannt, welche zur flächigen Behandlung von Knochendefekten zur Anwendung kommen. Häufig verfügt ein derartiges Implantat über eine Gitterstruktur, welche zumindest teilweise flexibel gestaltet ist und hierdurch eine Anpassung des Implantats an unterschiedliche Formen ermöglicht. Derartig gestaltete Implantate können im Bereich des Thorax und hierbei insbesondere bei Rippenfrakturen anstelle von Rippenplatten angewendet werden, um durch eine großflächige Anordnung am Thorax auch Bereiche zwischen verschiedenen Rippen abzudecken. Durch die Gitterstruktur wird dabei bei gleichzeitigem Schutz innenliegender Organe eine ausreichende Flexibilität verwirklicht, um eine ausreichende Beweglichkeit des Thorax zu ermöglichen.

Darüber hinaus werden Implantate mit Gitterstruktur im Bereich des Craniums für die Behandlung von Schädeldefekten vorgesehen, wobei die jeweilige Gitterstruktur dabei eine Anpassung des Implantats an gekrümmte Teile des Schädels ermöglicht und zudem eine ausreichende Elastizität sicherstellt. Üblicherweise verfügt ein Implantat zur flächigen Behandlung von Knochendefekten dabei über einen netzartigen, die Gitterstruktur bildenden Tragabschnitt, welcher am Knochen befestigt wird und der Stabilisierung dient.

Aus der DE 197 46 396 A1 geht ein Implantat hervor, welches für eine flächige Behandlung von Knochendefekten im Bereich des Craniums vorgesehen ist. Das Implantat weist eine Gitterstruktur auf, welche sich aus mehreren, in sich geschlossenen und untereinander verbundenen Segmenten zusammensetzt. Die Gitterstruktur kann an einer Unterseite zur Fixierung von Knochenteilen oder für eine Überbrückung von Knochenfehlstellen in dem entsprechenden Bereich des Craniums befestigt werden, wobei für die Befestigung dabei durch jedes einzelne Segment je eine Knochenschraube hindurchgeführt werden kann. Die untereinander verbundenen Segmente bilden dabei bei der Gitterstruktur einen zusammenhängenden Tragabschnitt, über welchen seitens der Knochenteile bzw. der Knochenfehlstelle eine Stabilisierung realisierbar ist. Die Gitterstruktur ist hierbei mittels Ätzen aus Titan hergestellt.

Ausgehend vom vorstehend beschriebenen Stand der Technik ist es nun die Aufgabe der vorliegenden Erfindung ein Implantat für eine flächige Behandlung von Knochendefekten zu schaffen, wobei komplexe Geometrien des Implantats bei niedrigem Herstellungsaufwand realisierbar sein sollen. Ferner sollen durch das erfindungsgemäße Implantat möglichst geringe Irritationen, insbesondere Gewebeirritationen, im Körper des jeweiligen Patienten hervorgerufen werden.

Diese Aufgabe wird ausgehend vom Oberbegriff des Anspruchs 1 in Verbindung mit dessen kennzeichnenden Merkmalen gelöst. Die hierauf folgenden, abhängigen Ansprüche geben jeweils vorteilhafte Weiterbildungen der Erfindung wieder. Weitere vorteilhafte Ausgestaltungen ergeben sich aus der Beschreibung sowie aus den Figuren.

Gemäß der Erfindung umfasst ein Implantat eine flexible Gitterstruktur mit einer Oberseite und einer der Oberseite abgewandt liegenden Unterseite, auf welcher eine knochenseitige Befestigung des Implantats vorzunehmen ist. Dabei weist die Gitterstruktur mindestens einen Tragabschnitt auf.

Das erfindungsgemäße Implantat ist dabei für eine flächige Behandlung eines Knochendefekts vorgesehen, wobei es sich dabei insbesondere um einen Knochendefekt im Bereich des Thorax oder des Craniums handelt. Für diese flächige Behandlung ist das Implantat mit einer Gitterstruktur ausgestattet, welche bevorzugt flächig gestaltet ist. Dabei verfügt die Gitterstruktur über eine Oberseite und eine Unterseite, welche einander abgewandt liegen und von denen es sich bei der Unterseite um die Seite handelt, mit welcher das Implantat auf den Knochendefekt zugewandt aufgelegt und befestigt wird.

Unter einer "flächigen" Behandlung ist im Sinne der Erfindung insbesondere zu verstehen, dass über das Implantat bei Anwendung an dem jeweiligen Knochendefekt eine Behandlungsfläche abgedeckt wird. Im Rahmen der Erfindung kann das Implantat konkret dafür vorgesehen sein, bei dieser flächigen Behandlung dabei Knochensegmente eines frakturierten Knochens zu verbinden und/oder Verbindungen zwischen unterschiedlichen Knochen herzustellen und/oder Knochenfehlstellen zu überdecken. Eine durch das erfindungsgemäße Implantat abzudeckende Behandlungsfläche kann dabei je nach konkretem Anwendungsfall, also insbesondere ob eine Anwendung im Bereich des Thorax oder des Craniums vorgesehen ist, im Bereich von wenigen Quadratmillimetern bis hin zu mehreren Quadratzentimetern liegen.

Die Gitterstruktur flexibel gestaltet, wodurch der Gitterstruktur eine 3-dimensionale Formbarkeit verliehen wird, um problemlos eine Anpassung der Form des Implantats an den jeweiligen Behandlungsbereich vornehmen zu können. So ist die Gitterstruktur des erfindungsgemäßen Implantats durch ihre flexible Gestaltung an gekrümmte Knochendefekte sowie Knochendefekte mit unregelmäßiger Form anpassbar. Ferner kann die Gitterstruktur im Rahmen der Erfindung dazu ausgestaltet sein, durch den jeweils behandelnden Chirurgen von ihrer Größe und Form her durch Entfernen von Teilen der Gitterstruktur angepasst zu werden, beispielsweise durch Zuschneiden der Gitterstruktur.

Erfindungsgemäß verfügt die Gitterstruktur über mindestens einen Tragabschnitt, der bevorzugt einer knochenseitigen Stabilisierung dient. Insbesondere ist dieser mindestens eine Tragabschnitt dabei dafür vorgesehen im Bereich des zu behandelnden Knochendefekts knochenseitig befestigt zu werden, d.h. mit mehreren Knochensegmenten eines Knochens bzw. mit einem oder mehreren Knochen bzw. -teilen verbunden zu werden. Die Stabilisierung über den mindestens einen Tragabschnitt ist insbesondere dadurch verwirklicht, dass dieser im angewendeten Zustand des Implantats eine oder mehrere knochenseitige Verbindungen herstellt. Bevorzugt ist der mindestens eine Tragabschnitt dazu mit mindestens einem Befestigungspunkt versehen, an dem die Befestigung des Implantats beispielsweise über je eine Knochenschraube realisierbar ist. Das erfindungsgemäße Implantat kann einen oder mehrere Tragabschnitte aufweisen.

Die Erfindung umfasst nun die technische Lehre, dass der mindestens eine Tragabschnitt mittels eines additiven Fertigungsverfahrens hergestellt worden ist. Des Weiteren ist auf den mindestens einen Tragabschnitt zumindest abschnittsweise eine Deckschicht aufgebracht, welche im Vergleich zu dem mindestens einen Tragabschnitt eine geringere Härte aufweist. Mit anderen Worten ist bei dem erfindungsgemäßen Implantat die Ausgestaltung des mindestens einen Tragabschnitt im Rahmen eines additiven Fertigungsverfahrens erfolgt. Zudem ist der mindestens eine Tragabschnitt zumindest abschnittsweise mit einer Deckschicht beschichtet, wobei diese Deckschicht dabei im Vergleich zu dem mindestens einen Tragabschnitt mit einer niedrigeren Härte ausgestaltet ist.

Eine derartige Ausgestaltung eines Implantats hat den Vorteil, dass der mindestens eine Tragabschnitt durch die additive Fertigung problemlos mit komplexen Geometrien gestaltet werden kann und dies dabei bei niedrigem Herstellungsaufwand möglich ist. So kann insbesondere eine komplexe und gleichzeitig flexible Struktur des mindestens einen Tragabschnitts und damit auch der Gitterstruktur verwirklicht werden. Durch das zumindest abschnittsweise Aufbringen der Deckschicht auf den mindestens einen Tragabschnitt und die im Vergleich zum Tragabschnitt geringere Härte dieser Deckschicht können Haut- und/oder Gewebeirritationen vermieden werden, welche ihre Ursache in der höheren Härte des mindestens einen Tragabschnitts und insbesondere aufgrund fertigungsbedingten Unebenheiten des mindestens einen Tragabschnitts haben. Denn durch das zumindest abschnittsweise Abdecken des mindestens einen Tragabschnitts mit der Deckschicht wird in dem jeweiligen Bereich aufgrund der niedrigeren Härte der Deckschicht eine weichere Oberfläche des Implantats verwirklicht. Insgesamt wird also ein Implantat realisiert, bei welchem mindestens ein Tragabschnitt einer Gitterstruktur auf geeignete Weise bei niedrigem Herstellungsaufwand ausgestaltet ist und bei dessen Verwendung das Auftreten von Haut- und/oder Gewebeirritationen wesentlich reduziert werden kann.

Wesentlich für die Erfindung ist, dass der mindestens eine Tragabschnitt des erfindungsgemäßen Implantats mittels eines additiven Fertigungsverfahrens ausgestaltet worden ist, wobei dies besonders bevorzugt im Rahmen eines 3D-Druckverfahrens erfolgt. Darüber hinaus ist der mindestens eine Tragabschnitt zumindest an Teilabschnitten mit der Deckschicht versehen, die im Vergleich zu dem mindestens einen Tragabschnitt weniger hart und damit weicher ausgeführt ist. Dadurch wird das erfindungsgemäße Implantat im Bereich der Beschichtung des mindestens einen Tragabschnitts mit der Deckschicht mit einer weicheren Oberfläche versehen.

Im Sinne der Erfindung ist unter "Härte" der mechanische Widerstand zu verstehen, der einem mechanischen Eindringen entgegengesetzt wird. Dementsprechend setzt die jeweils eine Deckschicht einem mechanischen Eindringen einen geringeren mechanischen Widerstand entgegen als dies bei dem mindestens einen Tragabschnitt der Fall ist. Die jeweils eine Deckschicht kann im Vergleich zu dem mindestens einen Tragabschnitt als weicher beschrieben werden.

Erfindungsgemäß ist der mindestens eine Tragabschnitt zumindest abschnittsweise mit der Deckschicht beschichtet, d.h. der mindestens eine Tragabschnitt kann also an einem oder mehreren Abschnitten oder auch vollumfänglich mit der Deckschicht versehen sein.

Entsprechend einer möglichen Ausführungsform ist der mindestens eine Tragabschnitt auf der Oberseite der Gitterstruktur an mindestens einem Teilabschnitt mit der Deckschicht beschichtet. In diesem Fall ist die Deckschicht also auf den mindestens einen Tragabschnitt abschnittsweise auf der Oberseite aufgebracht, wodurch der mindestens eine Tragabschnitt an der Oberseite abschnittsweise unbeschichtet ist. Alternativ dazu ist die Beschichtung des mindestens einen Tragabschnitts mit der Deckschicht vollständig auf der Oberseite der Gitterstruktur vorgenommen, sodass der mindestens eine Tragabschnitt an der Oberseite vollständig mit der Deckschicht abgedeckt ist. In vorteilhafter Weise ist der mindestens eine Tragabschnitt somit auf der Oberseite entweder in bestimmten Bereichen gezielt abgedeckt oder vollständig beschichtet. Im letztgenannten Fall werden dadurch Gewebeirritationen auf der Oberseite vollständig verhindert, während bei der abschnittsweisen Beschichtung gezielt Kontaktbereiche mit Gewebe und/oder Unebenheiten des Tragabschnitts, wie beispielsweise Kanten oder Ähnliches, mithilfe der Deckschicht weicher gestaltet werden können.

Alternativ oder ergänzend kann der mindestens eine Tragabschnitt auf der Unterseite der Gitterstruktur an mindestens einem Teilabschnitt mit der Deckschicht beschichtet sein. Der mindestens eine Tragabschnitt ist in diesem Fall an der Unterseite der Gitterstruktur abschnittsweise mit der Deckschicht versehen, wodurch der mindestens eine Tragabschnitt an der Unterseite abschnittsweise unbeschichtet ist. Der mindestens eine Tragabschnitt könnte aber auch auf der Unterseite der Gitterstruktur vollständig mit der Deckschicht beschichtet sein, womit der mindestens eine Tragabschnitt dann auf der Unterseite vollständig mit der Deckschicht abgedeckt ist. In beiden Fällen wird dadurch an der Unterseite des Implantats zumindest abschnittsweise eine weichere Oberfläche verwirklicht, indem der mindestens eine Tragabschnitt an der Unterseite entweder zumindest abschnittsweise oder vollständig mit der jeweils einen Deckschicht beschichtet ist. Hierdurch können Gewebeirritationen an der Unterseite des Implantats reduziert werden. Im Falle der abschnittsweisen Beschichtung des mindestens einen Tragabschnitts wird dieser dabei insbesondere gezielt in bestimmten Kontaktbereichen mit Gewebe und/oder an Unebenheiten, wie beispielsweise Kanten oder ähnlichem, beschichtet.

Die vorgenannten Weiterbildungen der Erfindung können dabei alternativ oder auch ergänzend verwirklicht sein, wodurch der mindestens eine Tragabschnitt abschnittsweise oder vollständig auf der Oberseite, abschnittsweise oder vollständig auf der Unterseite oder sowohl abschnittsweise oder vollständig auf der Oberseite, als auch abschnittsweise oder vollständig auf der Unterseite beschichtet sein kann.

Bei Kombination ist zudem eine Ausführung des Implantats denkbar, bei welcher der mindestens eine Tragabschnitt vollständig mit der jeweils einen Deckschicht ummantelt ist. In diesem Fall ist der mindestens eine Tragabschnitt dann also vollständig von der Deckschicht umschlossen, und damit vollständig beschichtet.

Gemäß einer Ausgestaltungsmöglichkeit der Erfindung weist der mindestens eine Tragabschnitt eine Netzstruktur auf, welche durch miteinander über Zwischensegmente verbundene, in sich geschlossene Segmente gebildet ist. Hierdurch wird ein geeigneter Aufbau erreicht, bei welchem eine hohe Beweglichkeit und damit Flexibilität im Bereich des mindestens einen Tragabschnitts realisiert werden kann. Bevorzugt sind die in sich geschlossenen Segmente ringförmig gestaltet, wobei die Segmente auch hiervon abweichende Gestaltungen aufweisen können, beispielsweise jeweils in Form je eines Polygons. Die Segmente sind bei der Netzstruktur über Zwischensegmente miteinander verbunden, die linear oder auch nichtlinear gestaltet sein können. Denkbar wäre zudem, dass die Zwischensegmente durch Befestigung aneinander je ein geschlossenes Segment definieren.

Im Rahmen der Erfindung kann der mindestens eine Tragabschnitt plattenförmig gestaltet sein. In diesem Fall weist der mindestens eine Tragabschnitt die Gestalt je einer Platte auf, wobei der mindestens eine Tragabschnitt dabei als steife Platte ausgeführt sein kann. Bei dem erfindungsgemäßen Implantat kann dadurch gezielt ein steifer Bereich definiert sein, in welchem eine besonders hohe Stabilität des Implantats zu realisieren ist. Die plattenförmige Gestaltung ist dabei insbesondere dann verwirklicht, wenn sich das erfindungsgemäße Implantat aus mehreren Tragabschnitten zusammensetzt, wobei dann einer oder mehrere der Tragabschnitte je eine Netzstruktur aufweisen und einer oder mehrere der Tragabschnitte plattenförmig ausgeführt sein können.

Es ist eine weitere mögliche Ausführungsform der Erfindung, dass die Deckschicht porös ausgebildet ist. Dies hat den Vorteil, dass durch diese poröse Gestaltung eine besonders niedrige Härte der jeweiligen Deckschicht erreicht und damit auch eine sehr weiche Oberfläche verwirklicht werden kann. Zum anderen wird hierdurch die Möglichkeit geschaffen, dass Gewebe in das Implantat einwachsen kann und eine Vaskularisierung möglich ist. Außerdem können hierdurch Körperflüssigkeiten durch die Deckschicht hindurchgelangen. In Weiterbildung dieser Ausführungsform ist der mindestens eine Tragabschnitt zumindest abschnittsweise in die poröse Deckschicht eingebettet. **In** vorteilhafter Weise kann hierdurch in dem entsprechenden Bereich eine ebenmäßige, weiche Oberfläche verwirklicht werden.

Alternativ dazu kann die Deckschicht nicht porös ausgeführt sein. Dadurch kann in diesem Bereich eine sehr glatte und zugleich weiche Oberfläche realisiert werden, wodurch Gewebeirritationen weitestgehend vermindert sind. Durch den nicht porösen Aufbau der jeweils einen Deckschicht kann ein Anwachsen von Geweben verhindert werden. Insbesondere entspricht hierbei eine Strukturierung der Deckschicht einer Strukturierung, welche der mindestens eine Tragabschnitt im Bereich der Beschichtung mit der jeweils einen Deckschicht aufweist. Dies hat den Vorteil, dass die Struktur des mindestens einen Tragabschnitts nach außen hin beibehalten wird.

Vorzugsweise besteht der mindestens eine Tragabschnitt aus einem ersten biokompatiblen Werkstoff. Die Deckschicht ist vorzugsweise aus einem zweiten biokompatiblen Werkstoff hergestellt, welcher eine geringere Härte aufweist als der erste Werkstoff. Hierdurch kann die im Vergleich zu dem mindestens einen Tragabschnitt geringere Härte der Deckschicht auf einfache Weise verwirklicht werden. Zudem können durch geeignete Wahl des zweiten Werkstoffs weitere geeignete Eigenschaften an der Oberfläche verwirklicht werden, wie beispielsweise die Ausgestaltung einer besonders glatten Oberfläche bei Verarbeitung des Werkstoffs.

In Weiterbildung der vorgenannten Ausgestaltungsmöglichkeit ist der erste Werkstoff ein Metall oder eine Metalllegierung, insbesondere Titan oder eine Titanlegierung. Ganz besonders bevorzugt handelt es sich bei dem ersten Werkstoff aber um einen Kunststoff, wobei dieser Kunststoff dabei insbesondere ein Polymer, bevorzugt ein Thermoplast und besonders bevorzugt Polyetheretherketon (PEEK) ist. Denn Polyetheretherketon zeichnet sich durch eine sehr gute Biokompatibilität und eine hohe erreichbare Festigkeit aus.

Der zweite Werkstoff ist vorzugsweise ein Kunststoff, insbesondere ein Polymer, bevorzugt ein Thermoplast und besonders bevorzugt Polyethylen (PE), beispielsweise ultrahochmolekulargewichtiges Polyethylen (UHMWPE) oder Polyethylen hoher Dichte (HDPE). Hierdurch kann die im Vergleich zu dem mindestens einen Tragabschnitt weichere Deckschicht zuverlässig hergestellt werden. Ferner kann hierdurch sowohl eine poröse Deckschicht durch Verwendung von PE-Granulat, als auch eine nicht poröse Deckschicht durch Verwendung von PE-Pulver verwirklicht werden, wobei sich im zweitgenannten Fall eine besonders glatte Oberfläche erreichen lässt.

Ganz besonders bevorzugt sind die beiden vorgenannten Varianten gemeinsam verwirklicht, wobei zur Herstellung des erfindungsgemäßen Implantats zunächst die additive Herstellung des mindestens einen Tragabschnitts aus Polyetheretherketon (PEEK) vorgenommen wird. Anschließend wird zur Reinigung bevorzugt eine Plasmabehandlung/Plasmaaktivierung des mindestens einen Tragabschnitts durchgeführt, bevor der mindestens eine Tragabschnitt gemeinsam mit dem PE-Pulver bzw. PE-Granulat in einer Negativform schichtweise platziert bzw. eingebettet wird. In einem darauffolgenden Pressvorgang wird das Polyethylen (PE) dann zusammen mit dem Polyetheretherketon (PEEK) erhitzt, wodurch das Polyethylen mit dem Polyetheretherketon verschmilzt. Hierdurch lässt sich ein belastbarer Verbund zwischen Polyetheretherketon und Polyethylen erreichen.

Im Rahmen der Erfindung könnten der mindestens eine Tragabschnitt und die jeweils eine aufgebrachte Deckschicht aber auch aus ein und demselben Werkstoff bestehen. So könnten sowohl der mindestens eine Tragabschnitt, als auch die Deckschicht beispielsweise jeweils aus Polyethylen hergestellt sein.

Entsprechend einer Ausführungsform der Erfindung weist die Gitterstruktur einen einzigen zusammenhängenden Tragabschnitt auf. In diesem Fall ist also die Gitterstruktur durch einen einzigen Tragabschnitt gebildet.

Alternativ dazu weist die Gitterstruktur mehrere Tragabschnitte auf, wobei benachbart liegende Tragabschnitte über jeweils einen zwischenliegenden Verbindungsabschnitt miteinander verbunden sind, der eine im Vergleich zu den benachbart liegenden Tragabschnitten geringere Steifigkeit aufweist. Dies hat den Vorteil, dass die Flexibilität der Gitterstruktur dadurch gezielt im Bereich des jeweiligen Verbindungsabschnitts erhöht werden kann. Denn aufgrund der niedrigeren Steifigkeit des jeweils zwischenliegenden Verbindungsabschnitts kann die Gitterstruktur und damit auch das Implantat in diesem Bereich leichter verformt werden. Zudem kann hierdurch auch mittels des mindestens einen zwischenliegenden Verbindungsabschnitts gezielt lokal die Gefahr von Haut- und/oder Gewebeirritationen reduziert werden.

Besonders bevorzugt ist die niedrigere Steifigkeit des jeweils zwischenliegenden Verbindungsabschnitts dadurch verwirklicht, indem der jeweils zwischenliegenden Verbindungsabschnitt aus einem biokompatiblen Werkstoff hergestellt ist, welcher eine geringere Werkstoffsteifigkeit aufweist als der Werkstoff des mindestens einen Tragabschnitts. Bei Ausgestaltung des mindestens einen Tragabschnitts und der Deckschicht aus unterschiedlichen Werkstoffen ist der jeweils zwischenliegende Verbindungsabschnitt insbesondere aus dem gleichen Werkstoff hergestellt wie die Deckschicht.

Im Rahmen der Erfindung kann der jeweils eine zwischenliegende Verbindungsabschnitt als lineares oder nichtlineares Zwischenstück oder auch in Form einer Netzstruktur vorliegen. Zudem könnte der jeweils eine zwischenliegende Verbindungsabschnitt auch plattenartig gestaltet sein.

Alternativ oder ergänzend dazu sind der jeweilige Tragabschnitt und der jeweils eine Verbindungsabschnitt stoffschlüssig miteinander verbunden. Dies ist dabei insbesondere dann verwirklicht, wenn der jeweils eine Verbindungsabschnitt und der jeweilige Tragabschnitt jeweils aus einem Kunststoffmaterial und hierbei insbesondere einem thermoplastischen Kunststoff bestehen.

Um die Verbindung zwischen dem jeweiligen Tragabschnitt und dem jeweils einen Verbindungsabschnitt robuster zu gestalten, ist es im Rahmen der Erfindung denkbar, dass der jeweilige Tragabschnitt und der jeweils eine Verbindungsabschnitt im Bereich der jeweiligen Befestigung in einer quer zu der Oberseite unter Unterseite verlaufenden Richtung überdecken. Dadurch wird die Belastbarkeit der Verbindung zwischen dem jeweiligen Tragabschnitt und dem jeweils einen Verbindungsabschnitt erhöht. Ganz besonders bevorzugt umgreift der jeweils eine Verbindungsabschnitt den jeweiligen Tragabschnitt im Bereich der jeweiligen Befestigung dabei beidseitig mit vorstehenden Verbindungssegmenten.

Vorteilhafte Ausführungsformen der Erfindung, die nachfolgend erläutert werden, sind in den Zeichnungen dargestellt. Es zeigen:
- Fig. 1: eine Draufsicht auf ein Implantat entsprechend einer ersten Ausführungsform;
- Fig. 2: eine schematische Schnittdarstellung des Implantats aus Fig. 1;
- Fig. 3: eine perspektivische Ansicht eines Implantats gemäß einer zweiten Ausgestaltungsmöglichkeit;
- Fig. 4 bis 6: schematische Darstellungen von Abwandlungsmöglichkeiten der Implantate aus den Fig. 1 und 3;
- Fig. 7: eine schematische Darstellung eines Implantats gemäß einer dritten Ausführungsform;
- Fig. 8: eine schematische Schnittdarstellung des Implantats aus Fig. 7; und
- Fig. 9 und 10: Teilansichten von Implantaten entsprechend weiterer Ausgestaltungsmöglichkeiten.

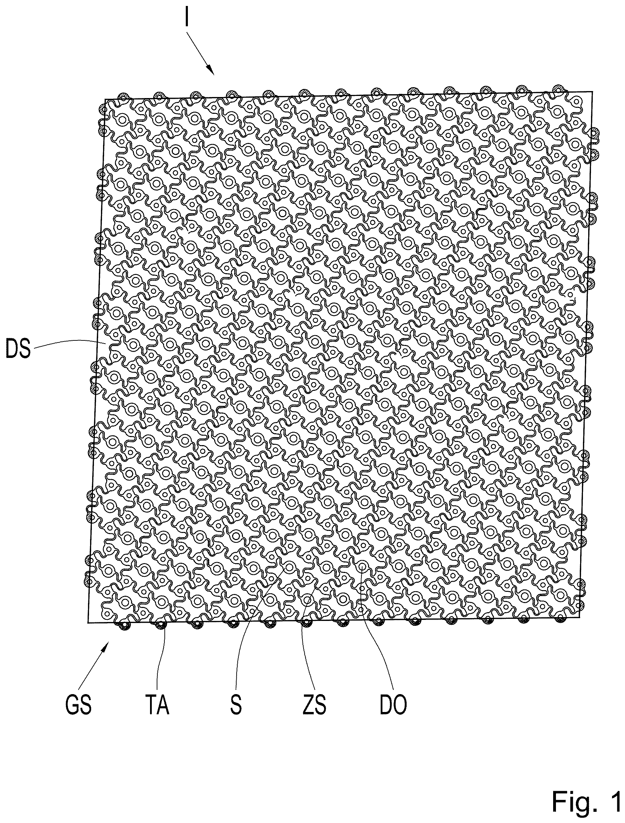

Aus Fig. 1 geht eine Draufsicht eines Implantats I hervor, welches für eine flächige Behandlung von Knochendefekten und hierbei insbesondere im Bereich des Thorax vorgesehen ist. Das Implantat I umfasst eine Gitterstruktur GS, welche vorliegend durch einen Tragabschnitt TA gebildet ist. Wie in Fig. 1 zu erkennen ist, weist dieser Tragabschnitt TA dabei eine Netzstruktur auf, indem sich der Tragabschnitt TA aus ringförmigen Segmenten S und Zwischensegmenten ZS zusammensetzt, über welche die ringförmigen Segmente S untereinander verbunden sind. Die Zwischensegmente ZS sind dabei linearförmig verlaufend gestaltet.

Bei dem Tragabschnitt TA bilden die ringförmigen Segmente S jeweils eine Durchgangsöffnung DO, in welcher je eine Knochenschraube zur Befestigung des Implantats I aufgenommen werden kann, wobei über hindurchgeführte Knochenschrauben eine knochenseitige Befestigung des Implantats I im Bereich des zu behandelnden Knochendefekts hergestellt werden kann. So können mithilfe des Tragabschnitts TA im Bereich des Thorax Knochensegmente bzw. Knochenteile eines oder mehrerer Rippenknochen und/oder mehrere Rippenknochen untereinander verbunden werden, wobei der Tragabschnitt TA dabei eine Stabilisierung der Knochensegmente bzw. Knochenteile zueinander und/oder der Rippenknochen zueinander verwirklicht.

Die Zwischensegmente ZS ermöglichen Bewegungen der ringförmigen Segmente S zueinander, wodurch die Netzstruktur des Tragabschnitts TA insgesamt flexibel gestaltet ist. Dies verleiht der Gitterstruktur GS des Implantats I insgesamt eine flexible Eigenschaft und ermöglicht zum einen eine Anpassung an gekrümmte Verläufe der Knochen im Bereich des zu behandelnden Knochendefekts. Zum anderen wird dadurch im befestigten Zustand des Implantats I eine gewisse Beweglichkeit im Bereich des zu behandelnden Knochendefekts zugelassen, um Bewegungen des Thorax aufgrund der Atmung oder von Bewegungen des Patienten zu ermöglichen.

Der Tragabschnitt TA ist vorliegend aus Polyetheretherketon (PEEK) im Rahmen eines additiven Fertigungsverfahrens hergestellt worden, wobei der Tragabschnitt TA hierbei konkret im 3D-Druckverfahren ausgestaltet wurde. Hierdurch lässt sich die komplexe Netzstruktur des Tragabschnitts TA mit niedrigem Herstellungsaufwand fertigen, wobei sich über den biokompatiblen Werkstoff Polyetheretherketon (PEEK) zudem eine hohe Stabilität des Tragabschnitts TA verwirklichen lässt.

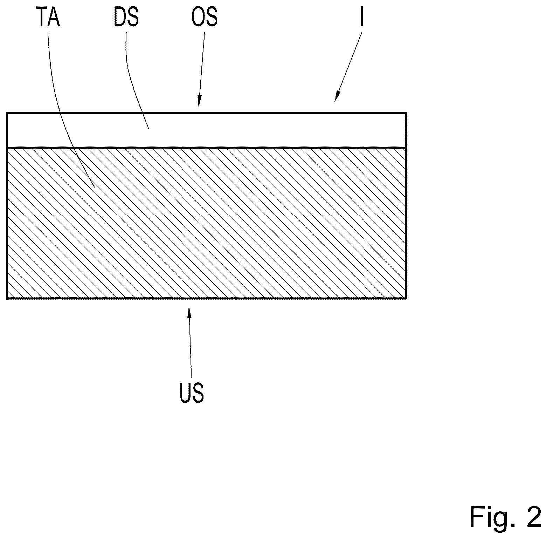

Allerdings hat die additive Herstellung des Tragabschnitts TA zur Folge, dass an dem Tragabschnitt TA eine raue Oberfläche und teilweise auch harte Kanten entstehen, was nach Platzierung des Implantats im Körper des jeweiligen Patienten zu entsprechenden Gewebeirritationen führen kann. Darüber hinaus ist die Netzstruktur des Tragabschnitts TA unter Umständen durch das Gewebe bzw. die Haut des Patienten hindurch spürbar, was ebenfalls in entsprechenden Irritationen resultieren kann. Um die Gefahr derartiger Irritationen zu reduzieren, ist der Tragabschnitt TA teilweise mit einer Deckschicht DS versehen, die im Vergleich zu dem Tragabschnitt TA eine geringere Härte aufweist und dementsprechend weicher gestaltet ist. Wie in Zusammenschau von Fig. 1 mit der schematischen Schnittansicht in Fig. 2 zu erkennen ist, ist diese Deckschicht DS dabei auf den Tragabschnitt TA auf einer Oberseite OS aufgebracht, welche bei dem Implantat I einer Unterseite US abgewandt liegt, an der das Implantat I knochenseitig befestigt wird.

Die Deckschicht DS besteht vorliegend aus ultrahochmolekulargewichtigen Polyethylen (UHMWPE), welches als biokompatibler Werkstoff im Vergleich zu dem Polyetheretherketon (PEEK) des Tragabschnitts TA eine geringere Härte aufweist. Die Deckschicht DS ist bei dem Implantat I plattenartig und porös gestaltet, wobei der Tragabschnitt TA dabei auf der Oberseite OS des Implantats I in die Deckschicht DS eingebettet ist. Aufgrund der porösen Gestaltung der Deckschicht DS wird hier eine besonders weiche Oberfläche an der Oberseite OS des Implantats I geschaffen, wobei zudem ein Einwachsen von Gewebe stattfinden kann und ein Hindurchgelangen von Körperflüssigkeiten möglich ist. Zudem kann hierdurch eine Vaskularisierung im Bereich des Implantats I stattfinden und Weichgewebe besser erhalten bleiben.

Für die Herstellung des Implantats I wurde der Tragabschnitt TA der Gitterstruktur GS nach seiner additiven Fertigung zur Reinigung einer Plasmabehandlung/Plasmaaktivierung unterzogen, wobei der Tragabschnitt TA im Anschluss daran gemeinsam mit Polyethylen-Granulat schichtweise in einer Negativform platziert wurde. Daraufhin wurde dann das Implantat I dadurch ausgebildet, dass das Polyethylen gemeinsam mit dem Polyetheretherketon im Rahmen eines Pressvorgangs erhitzt und im Zuge dessen das Polyethylen mit dem Polyetheretherketon verschmolzen wurde.

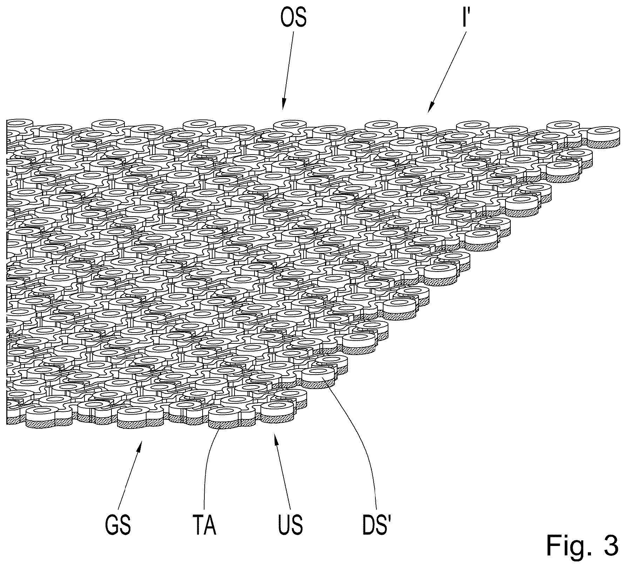

Fig. 3 zeigt eine perspektivische Ansicht eines Implantats I' gemäß einer zweiten Ausgestaltungsmöglichkeit der Erfindung. Dabei entspricht dieses Implantat I' weitestgehend der vorhergehenden Variante nach den Fig. 1 und 2. So weist auch das Implantat I' eine Gitterstruktur GS auf, welche durch einen eine Netzstruktur aufweisenden und additiv hergestellten Tragabschnitt TA aus Polyetheretherketon (PEEK) gebildet ist. Ferner ist der Tragabschnitt TA auch bei dem Implantat l' abschnittsweise beschichtet, indem auf den Tragabschnitt TA auf einer Oberseite OS des Implantats I' eine Deckschicht DS' aufgebracht ist. Die Deckschicht DS' ist dabei in Übereinstimmung mit der Variante nach den Fig. 1 und 2 aus Polyethylen (PE) hergestellt, wobei die Deckschicht DS' nun aber im Unterschied zu dem Implantat I aus den Fig. 1 und 2 nicht porös ausgestaltet ist, wozu bei der Herstellung des Implantats I' Polyethylen-Pulver anstelle Polyethylen-Granulat verwendet wurde. Im Vergleich zu dem Implantat I aus den Fig. 1 und 2 wird dadurch eine glatte Oberfläche des Implantats I' auf der Oberseite OS erreicht. Wie zudem in Fig. 3 zu erkennen ist, ist die Deckschicht DS' dabei mit einer Strukturierung ausgeführt, welche der Netzstruktur des darunterliegenden Tragabschnitts TA entspricht. Dadurch wird auch bei der nicht porösen Ausführung der Deckschicht DS' ein Einwachsen von Gewebe sowie ein Hindurchgelangen von Körperflüssigkeiten ermöglicht. Im Übrigen entspricht das Implantat I' dem Implantat I aus den Fig. 1 und 2, so dass auf das hierzu Beschriebene Bezug genommen wird.

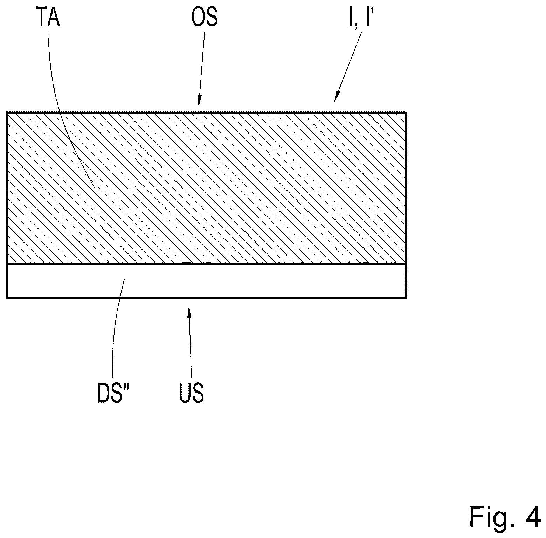

Die Fig. 4 bis 6 zeigen jeweils Abwandlungsmöglichkeiten der beiden Implantate I und I' nach den Fig. 1 bis 3. So ist der Tragabschnitt TA des jeweiligen Implantats I bzw. I' bei der Abwandlungsmöglichkeit nach Fig. 4 dadurch teilweise beschichtet, dass nun eine Deckschicht DS" auf der Unterseite US des Implantats I bzw. I' vorgesehen ist. Diese Deckschicht DS" ist dabei erneut im Vergleich zum Tragabschnitt TA mit einer geringeren Härte ausgestattet, indem die Deckschicht DS" aus Polyethylen (PE) gebildet ist. Zudem kann die Deckschicht DS" in analoger Weise zu dem vorstehend Beschriebenen porös und plattenartig oder nicht porös und mit einer der Netzstruktur des Tragabschnitts TA entsprechenden Strukturierung versehen sein.

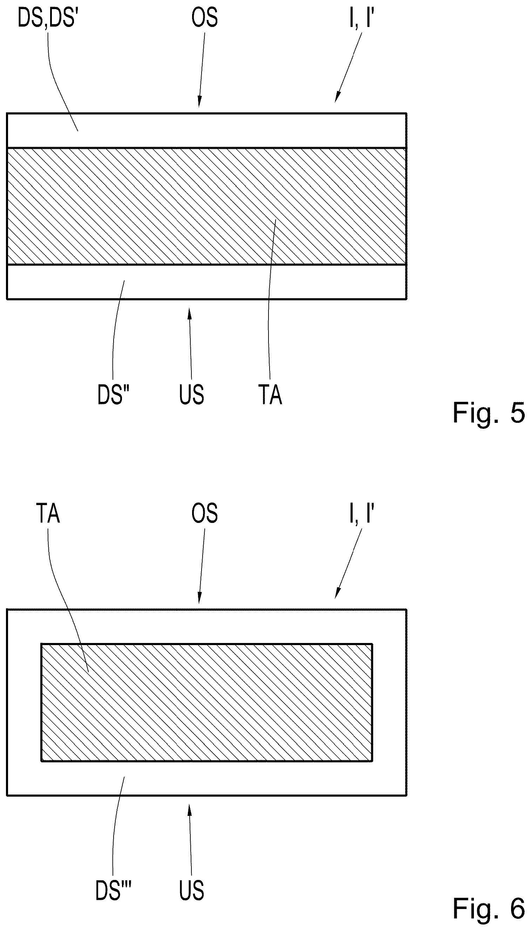

Bei der Abwandlungsmöglichkeit nach Fig. 5 ist das Implantat I bzw. I' hingegen an seinem Tragabschnitt TA sowohl auf der Oberseite OS mit der Deckschicht DS bzw. DS' sowie auf der Unterseite US mit der Deckschicht DS" beschichtet, wodurch der Tragabschnitt TA sandwichartig zwischen den Deckschichten DS bzw. DS' und DS" eingefasst ist.

Fig. 6 zeigt eine Abwandlungsmöglichkeit der beiden Implantate I und I' nach den Fig. 1 bis 3, wobei in diesem Fall der Tragabschnitt TA vollständig mit einer Deckschicht DS‴ ummantelt ist. Prinzipiell könnte auch diese Deckschicht DS‴ dabei porös oder nicht porös ausgeführt sein.

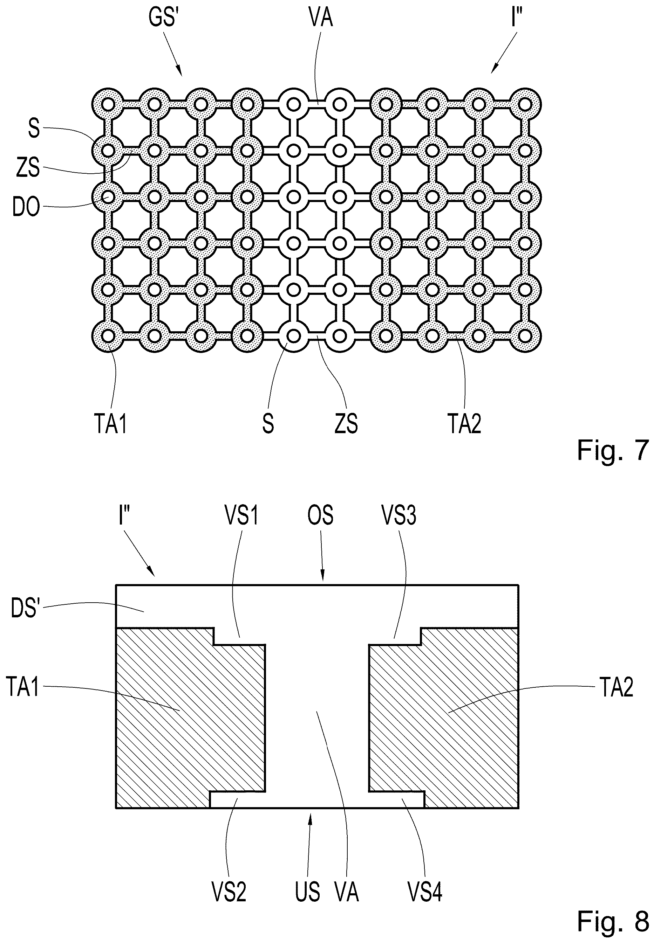

Aus Fig. 7 geht eine schematische Ansicht eines Implantats I" gemäß einer weiteren Ausführungsform der Erfindung hervor, wobei dieses Implantat I" dabei im Wesentlichen dem Implantat I' aus Fig. 3 entspricht. Im Unterschied zu dem Implantat I' ist bei dem Implantat I" eine Gitterstruktur GS' nun nicht durch einen einzigen Tragabschnitt, sondern durch mehrere Tragabschnitte TA1 und TA2 gebildet, welche jeweils eine Netzstruktur aufweisen. Dabei ist die jeweilige Netzstruktur des jeweiligen Tragabschnitts TA1 bzw. TA2 erneut durch ringförmige Segmente S gebildet, welche Durchgangsöffnungen DO für Knochenschrauben bilden und über Zwischensegmente ZS miteinander verbunden sind.

Die Tragabschnitte TA1 und TA2 sind zur Bildung der Gitterstruktur GS' über jeweils einen zwischenliegenden Verbindungsabschnitt VA miteinander verbunden, wobei dieser Verbindungsabschnitt VA im Vergleich zu den Tragabschnitten TA1 und TA2 mit einer geringeren Steifigkeit ausgeführt ist. Dadurch wird im Vergleich zu dem Implantat I' aus Fig. 3 eine höhere Flexibilität des Implantats I" erreicht.

Auch der Verbindungsabschnitt VA weist in Übereinstimmung mit den Tragabschnitten TA1 und TA2 eine Netzstruktur aus Segmenten S und Zwischensegmenten ZS auf, wobei die niedrigere Steifigkeit dabei dadurch erreicht ist, dass der Verbindungsabschnitt VA aus Polyethylen (PE) gebildet ist. Im Zuge der Fertigung des Implantats I" ist bei dem Pressvorgang eine stoffschlüssige Verbindung zwischen dem jeweiligen Tragabschnitts TA1 bzw. TA2 mit dem zwischenliegenden Verbindungsabschnitt VA ausgestaltet worden. Um die Belastbarkeit der jeweiligen Verbindung weiter zu erhöhen, überdeckt der zwischenliegende Verbindungsabschnitt VA, wie anhand der schematischen Darstellung des Implantats I" in Fig. 8 ersichtlich ist, jeweils im Bereich der jeweiligen Verbindung mit dem jeweiligen Tragabschnitt TA1 bzw. TA2. Dazu ist der Verbindungsabschnitt VA mit Verbindungssegmenten VS1 bis VS4 ausgestattet, welche an dem Verbindungsabschnitt VA in einer quer zu Oberseite OS und Unterseite US verlaufenden Richtung vorstehend ausgestaltet sind und mit denen der Verbindungsabschnitt VA den jeweiligen Tragabschnitt TA1 bzw. TA2 in dem jeweiligen Verbindungsbereich beidseitig umgreift.

In analoger Weise zu der Variante nach Fig. 3 ist zudem eine Deckschicht DS' vorgesehen, mit welcher bei dem Implantat I" nun die Tragabschnitte TA1 und TA2 und auch der Verbindungsabschnitt VA auf der Oberseite OS beschichtet sind. Die Deckschicht DS' ist als nicht poröse Deckschicht ausgestaltet. Im Übrigen entspricht das Implantat I" sonst der Variante nach Fig. 3, so dass auf das hierzu Beschriebene Bezug genommen wird. Zudem könnten auch bei dem Implantat I" die Abwandlungen gemäß der Fig. 4 bis 6 realisiert sein.

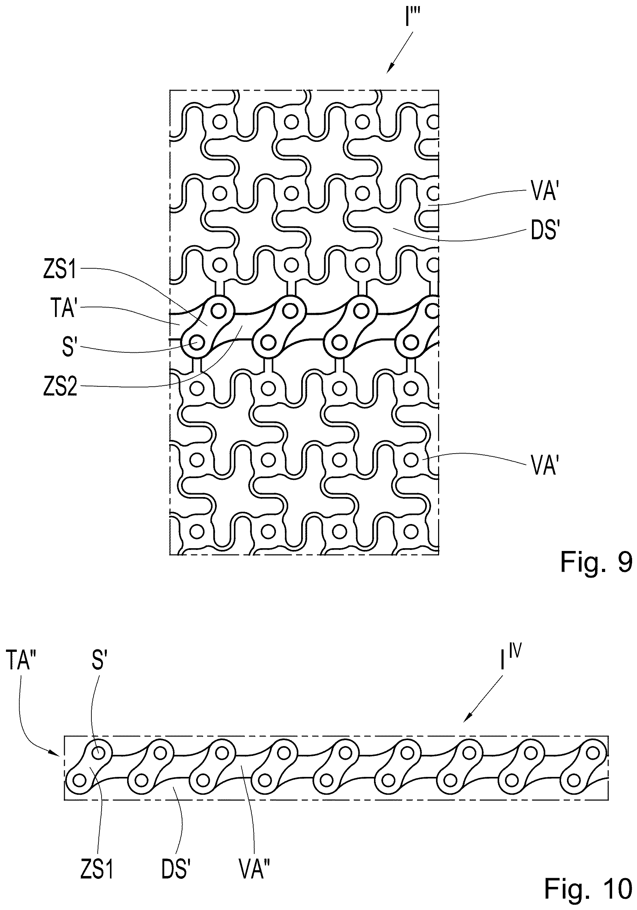

Fig. 9 zeigt einen Teil einer weiteren möglichen Ausgestaltung eines Implantats I‴, welches dabei weitestgehend der vorhergehenden Variante nach den Fig. 7 und 8 entspricht. So umfasst dieses Implantat I‴ ebenfalls mehrere Tragabschnitte TA', von denen in Fig. 9 allerdings lediglich ein Tragabschnitt TA' zu sehen ist. Im Unterschied zu dem Implantat I" aus den Fig. 7 und 8 ist der jeweilige Tragabschnitt TA' dabei allerdings plattenartig und steif aus Polyetheretherketon (PEEK) gestaltet. Dabei sind ringförmige Segmente S' einerseits über Zwischensegmente ZS1 paarweise miteinander verbunden und bilden hierdurch 8-förmige Gebilde, wobei diese Gebilde dabei dann über weitere, stegartige Zwischensegmente ZS2 untereinander unter Bildung des jeweiligen Tragabschnitts TA' miteinander in Verbindung stehen.

Zudem ist der jeweilige Tragabschnitt TA' mit benachbarten Tragabschnitten über zwischenliegende Verbindungsabschnitte VA' verbunden, welche jeweils aus Polyethylen (PE) bestehen. Eine Verbindung des einzelnen Verbindungsabschnitts VA' zu dem jeweiligen Tragabschnitt TA' ist dabei in analoger Weise zu der Variante nach Fig. 7 und 8 verwirklicht. Außerdem sind die Tragabschnitte TA' und die Verbindungsabschnitte VA' auch bei dem Implantat I‴ mit einer Deckschicht DS' versehen, wobei weitere Abwandlungen im Sinne einer der Varianten nach den Fig. 4 bis 6 ebenfalls denkbar sind. Auch ansonsten entspricht die Ausführungsform nach Fig. 9 der Variante nach den Fig. 7 und 8, so dass auf das hierzu Beschriebene Bezug genommen wird.

Schließlich zeigt noch Fig. 10 eine Ausgestaltung eines Implantats I^{IV}, wobei dieses Implantat I^{IV} dabei weitestgehend der vorhergehenden Variante nach Fig. 9 entspricht. Unterschiedlich ist dabei, dass bei dem Implantat I^{IV} nun Tragabschnitte TA" aus Polyetheretherketon (PEEK) als 8-förmige Gebilde ausgeführt sind, bei welchen ringförmige Segmente S' paarweise über je ein zwischenliegendes Zwischensegment ZS1 miteinander verbunden sind. Untereinander sind die Tragabschnitte TA" dann über Verbindungsabschnitte VA" verbunden, welche im Vergleich zu den Tragabschnitten TA" jeweils eine geringere Steifigkeit aufweisen, indem diese Verbindungsabschnitte VA" aus Polyethylen (PE) bestehen. Eine Verbindung des einzelnen Verbindungsabschnitts VA" mit dem jeweiligen Tragabschnitt TA" ist dabei in analoger Weise zu der Variante zu den Fig. 7 und 8 vollzogen, wobei die Tragabschnitte TA" und die Verbindungsabschnitte VA" zudem mit einer Deckschicht DS' beschichtet sind. Auch hier kann zudem eine weitere Abwandlung im Sinne einer der Varianten nach den Fig. 4 bis 6 realisiert sein.

Mittels der erfindungsgemäßen Ausgestaltungen kann jeweils ein Implantat für eine flächige Behandlung von Knochendefekten mit niedrigem Herstellungsaufwand geschaffen werden, wobei bei Anwendung dieses Implantats geringe Gewebeirritationen im Körper des jeweiligen Patienten hervorgerufen werden.

### Bezugszeichenliste

- I, I', I", I‴, I^{IV}: Implantat
- GS, GS': Gitterstruktur
- TA, TA1, TA2, TA', TA": Tragabschnitt
- S, S': Segment
- ZS, ZS1, ZS2: Zwischensegment
- DO: Durchgangsöffnung
- DS, DS', DS", DS‴: Deckschicht
- OS: Oberseite
- US: Unterseite
- VA, VA', VA": Verbindungsabschnitt
- VS1, VS2, VS3, VS4: Verbindungssegment

## Patentansprüche

1. Implantat (I; I'; I"; I‴; I^{IV}) für eine flächige Behandlung eines Knochendefekts, insbesondere eines Knochendefekts im Bereich des Thorax oder des Craniums, umfassend eine flexible Gitterstruktur (GS; GS') mit einer Oberseite (OS) und einer der Oberseite (OS) abgewandt liegenden Unterseite (US), auf welcher eine knochenseitige Befestigung des Implantats (I; I'; I"; I‴; I^{IV}) vorzunehmen ist, wobei die Gitterstruktur (GS; GS') mindestens einen Tragabschnitt (TA; TA1, TA2; TA'; TA") aufweist, **dadurch gekennzeichnet, dass** der mindestens eine Tragabschnitt (TA; TA1, TA2; TA'; TA") mittels eines additiven Fertigungsverfahrens hergestellt worden ist, und dass auf den mindestens einen Tragabschnitt (TA; TA1, TA2; TA'; TA") zumindest abschnittsweise eine Deckschicht (DS; DS'; DS"; DS‴) aufgebracht ist, welche im Vergleich zu dem mindestens einen Tragabschnitt (TA; TA1, TA2; TA'; TA") eine geringere Härte aufweist.

2. Implantat (I; I'; I"; I"'; I^{IV}) nach Anspruch 1, **dadurch gekennzeichnet, dass** der mindestens eine Tragabschnitt (TA; TA1, TA2; TA'; TA") auf der Oberseite (OS) der Gitterstruktur (GS; GS') an mindestens einem Teilabschnitt oder vollständig mit der Deckschicht (DS; DS'; DS‴) beschichtet ist.

3. Implantat (I; I'; I"; I‴; I^{IV}) nach Anspruch 1 oder 2, **dadurch gekennzeichnet, dass** der mindestens eine Tragabschnitt (TA; TA1, TA2; TA'; TA") auf der Unterseite (US) der Gitterstruktur (GS; GS') an mindestens einem Teilabschnitt oder vollständig mit der Deckschicht (DS"; DS‴) beschichtet ist.

4. Implantat (I; I'; I"; I‴; I^{IV}) nach den Ansprüchen 2 und 3, **dadurch gekennzeichnet, dass** der mindestens eine Tragabschnitt (TA; TA1, TA2; TA'; TA") vollständig mit der Deckschicht (DS"') ummantelt ist.

5. Implantat (I; I'; I") nach wenigstens einem der vorhergehenden Ansprüche, **dadurch gekennzeichnet, dass** der mindestens eine Tragabschnitt (TA; TA1, TA2) eine Netzstruktur aufweist, welche durch in sich geschlossene Segmente (S) gebildet ist, wobei die Segmente (S) durch Zwischensegmente (ZS) miteinander verbunden sind.

6. Implantat (I"'; I^{IV}) nach wenigstens einem der vorhergehenden Ansprüche, **dadurch gekennzeichnet, dass** der mindestens eine Tragabschnitt (TA'; TA") plattenförmig gestaltet ist.

7. Implantat (I; I"; I‴; I^{IV}) nach wenigstens einem der vorhergehenden Ansprüche, **dadurch gekennzeichnet, dass** die Deckschicht (DS; DS"; DS‴) porös ausgebildet ist.

8. Implantat (I; I"; I‴; I^{IV}) nach Anspruch 7, **dadurch gekennzeichnet, dass** der mindestens eine Tragabschnitt (TA; TA1, TA2; TA'; TA") zumindest abschnittsweise in die poröse Deckschicht (DS; DS"; DS"') eingebettet ist.

9. Implantat (I'; I"; I‴; I^{IV}) nach wenigstens einem der Ansprüche 1 bis 6, **dadurch gekennzeichnet, dass** die Deckschicht (DS'; DS"; DS‴) nicht porös ausgeführt ist.

10. Implantat (I'; I"; I"'; I^{IV}) nach Anspruch 9, **dadurch gekennzeichnet, dass** eine Strukturierung der Deckschicht (DS'; DS"; DS‴) einer Strukturierung entspricht, welche der mindestens eine Tragabschnitt (TA; TA1, TA2; TA'; TA") im Bereich der Beschichtung mit der jeweils einen Deckschicht (DS'; DS"; DS'") aufweist.

11. Implantat (I; I'; I"; I‴; I^{IV}) nach wenigstens einem der vorhergehenden Ansprüche, **dadurch gekennzeichnet, dass** der mindestens eine Tragabschnitt (TA; TA1, TA2; TA'; TA") aus einem ersten biokompatiblen Werkstoff besteht, wohingegen die Deckschicht (DS; DS'; DS"; DS‴) aus einem zweiten biokompatiblen Werkstoff hergestellt ist, welcher eine geringere Härte aufweist als der erste Werkstoff.

12. Implantat nach Anspruch 11, **dadurch gekennzeichnet, dass** der erste Werkstoff ein Metall oder eine Metalllegierung, insbesondere Titan oder eine Titanlegierung, oder ein Kunststoff ist, insbesondere ein Polymer, bevorzugt ein Thermoplast und besonders bevorzugt Polyetheretherketon (PEEK).

13. Implantat nach Anspruch 11 oder 12, **dadurch gekennzeichnet, dass** der zweite Werkstoff ein Kunststoff ist, insbesondere ein Polymer, bevorzugt ein Thermoplast und besonders bevorzugt Polyethylen (PE).

14. Implantat (I; I') nach wenigstens einem der vorhergehenden Ansprüche, **dadurch gekennzeichnet, dass** die Gitterstruktur (GS) einen einzigen zusammenhängenden Tragabschnitt (TA) aufweist.

15. Implantat (I"; I‴; I^{IV}) nach wenigstens einem der Ansprüche 1 bis 13, **dadurch gekennzeichnet, dass** die Gitterstruktur (GS') mehrere Tragabschnitte (TA1, TA2; TA'; TA") aufweist, wobei benachbart liegende Tragabschnitte (TA1, TA2; TA'; TA") über jeweils einen zwischenliegenden Verbindungsabschnitt (VA, VA', VA") miteinander verbunden sind, der jeweils eine im Vergleich zu den benachbart liegenden Tragabschnitten (TA1, TA2; TA'; TA") geringere Steifigkeit aufweist.
